# EUROPEAN PATENT SPECIFICATION

(11) **EP 4 033 986 B1**
(45) Date of publication and mention of the grant of the patent: **06.11.2024**
(21) Application number: 20768628.8
(22) Date of filing: 16.09.2020
(51) Int. Cl.: A61B 8/04, A61B 8/06, A61B 8/08, G16H 50/50

(54) **METHODS AND SYSTEMS FOR MODELING A CARDIAC SYSTEM**
VERFAHREN UND SYSTEME ZUR MODELLIERUNG EINES HERZSYSTEMS
PROCÉDÉS ET SYSTÈMES DE MODÉLISATION D'UN SYSTÈME CARDIAQUE

(30) Priority: 26.09.2019 US 201962906172 P; 10.10.2019 EP 19202538
(43) Date of publication of application: 03.08.2022
(73) Proprietor: Koninklijke Philips N.V., 5656 AG Eindhoven (NL)
(72) Inventor: LAU, Kevin, Daniel, Seng, Hung, 5656 AE Eindhoven (NL); SCHRAUWEN, Jelle, 5656 AE Eindhoven (NL); HAAK, Alexander, 5656 AE Eindhoven (NL); BARAGONA, Marco, 5656 AE Eindhoven (NL); MAESSEN, Ralph, Theodorus, Hubertus, 5656 AE Eindhoven (NL)
(74) Representative: Philips Intellectual Property & Standards
(86) International application number: PCT/EP2020/075792
(87) International publication number: WO 2021/058339

(56) References cited:
- US-A1- 2012 232 853
- US-A1- 2018 174 068
- TSALIKAKIS D G ET AL: "Simulation of cardiovascular diseases using electronic circuits", COMPUTERS IN CARDIOLOGY 2003. THESSALONIKI, GREECE, SEPT. 21 - 24, 2003; [COMPUTERS IN CARDIOLOGY], NEW YORK, NY : IEEE, US, vol. VOL. 30, 21 September 2003 (2003-09-21), pages 445 - 448, XP010698937, ISBN: 978-0-7803-8170-4, DOI: 10.1109/CIC.2003.1291188
- NEIL A TENENHOLTZ ET AL: "On the design of an interactive, patient-specific surgical simulator for mitral valve repair", INTELLIGENT ROBOTS AND SYSTEMS (IROS), 2011 IEEE/RSJ INTERNATIONAL CONFERENCE ON, IEEE, 25 September 2011 (2011-09-25), pages 1327 - 1332, XP032060830, ISBN: 978-1-61284-454-1, DOI: 10.1109/IROS.2011.6048851

## Description

### FIELD OF THE INVENTION

The invention relates to the field of cardiac system modelling, and more specifically to the field of hemodynamic function modelling.

### BACKGROUND OF THE INVENTION

The human heart contains four heart valves, aortic, mitral (MV), pulmonary and tricuspid. Located between the left atrium and left ventricle, the MV has two key functions: maintaining a hemodynamic seal during ventricular ejection; and ensuring rapid ventricular re-filling following ventricular ejection. Optimal MV function depends upon several factors, such as the hemodynamic load, the biomechanical properties of the MV connected tissue and the functional anatomy of the left heart. Dysfunction of one or more of these factors may result in suboptimal filling or ejection.

Mitral valve regurgitation (MVR) is the most common MV dysfunction and accounts for approximately 70% of native MV dysfunctions. Due to changes in MV function (such as MV prolapse or cardiomyopathy), the MV no longer maintains unidirectional flow in MVR. During ventricular ejection retrograde flow enters the left atrium. This increases the hemodynamic load in the left heart (also referred to as volume overload), which can lead to other cardiac related pathologies, such as atrial fibrillation.

MVR may be corrected by either: repairing the existing valve; or prosthetic replacement. However, repair has significant benefits, such as reduced mortality rates (2.0% repair, 6.1% replacement) and minimization of other complications such as thromboses. One such repair technique for MVR is the edge-to-edge repair (ETER), which attempts to restore MV coaptation by physically joining prolapsing regions together.

The ETER was originally developed as an open chest surgery (i.e. midline sternotomy). However, minimally invasive approaches are now available whereby the repair is performed using a percutaneously delivered device. Such a device may be in the form of a clip, which brings the prolapsing regions together. However, during deployment of such devices, there remains some uncertainty in the number of devices required to restore proper function of the valve. Typically, during the procedure the clinician must determine if the current repair is sufficient, or if the subject requires further repair.

Although the ETER alleviates MVR, it may negatively affect ventricular filling. Accordingly, the ETER aims to find a balance between reducing retrograde flow into the atrium during ventricular contraction and avoiding a reduction in orifice area that would impair ventricular filling. For this reason a dilemma typically encountered is the decision between placing an additional repair device (thereby further reducing the orifice area and possibly impairing ventricular filling) or accepting the current configuration with the risk of having insufficiently reduced the MVR.

Imaging tools may be used to provide anatomical guidance to reduce the uncertainty of the decision to provide an additional repair device. However, these tools lack hemodynamic information relating to the flow of blood in the region of the repair.

Document US 2018/174068 discloses a subject-specific simulation model of at least one component in the cardiovascular system for simulating blood flow and/or structural features.

In the scientific publication "Simulation of cardiovascular diseases using electronic circuits", Tsalikakis D.G. et al, Computers in Cardiology, Vol. 30, 2003, it is shown how a 0D numerical model might be used to simulate a cardiac system.

### SUMMARY OF THE INVENTION

The invention is defined by the claims.

According to an aspect of the invention, there is provided a system for determining a real-time valve function of a subject according to claim 1.

The system provides for a means of determining the real-time hemodynamic function of a subject.

For example, when undergoing percutaneous valvular repair, a clinician may require data relating to the hemodynamic function of a subject in order to make a decision. Such data is typically determined based upon qualitative anatomic inspection and user dependent measurements, both of which can vary in accuracy.

However, these decisions and their outcomes often remain unclear. Accordingly, by providing an accurate model of the real-time hemodynamic function of the subject, based on subject-specific measurements, it is possible to generate a more accurate depiction of the hemodynamic function of the subject over time. This may then lead to a clearer insight into the status of the cardiac system and subsequently to a more beneficial decision by the clinician.

In an embodiment, the continuous stream of physiological data is obtained from a subject undergoing a change in valve function, and wherein the real-time valve function determined from the simulated real-time function of the cardiac system is representative of the change in valve function.

In this way, the change in valve function may be monitored, or even predicted, by the system taking the entire cardiac system into account by way of the numerical model.

In a further embodiment, the system is adapted for use while the subject is undergoing a valve repair.

In this way, the system may provide information for guidance and support to a user during the process of repairing a valve.

In an embodiment, the system further comprises a physiological sensor adapted to obtain physiological data from the subject, wherein the physiological sensor comprises one or more of:
an electrocardiogram sensor, wherein the physiological data comprises electrocardiogram data;
a blood pressure measurement device, wherein the physiological data comprises numerical pressure data and/or pressure waveform data; and
a volume waveform sensor, wherein the physiological data comprises volume waveform data.

In a further embodiment, the volume waveform sensor comprises one or more of
an ultrasound transducer, wherein the volume waveform data comprises ultrasound data;
an inflatable cuff, adapted to be worn by the subject; and
a thermistor-tipped catheter, wherein the volume waveform data is derived using a thermodilution technique.

In an embodiment, the numerical model is based on a physical parameter, and wherein the processor is further adapted to adjust the physical parameter of the numerical model based on at least a portion of the continuous stream of physiological data.

In this way, the numerical model may be tailored to the subject according to variances in the physiological data over time.

In an embodiment, the numerical model is based on a physical parameter, and wherein the processor is further adapted to:
obtain preliminary physiological data from the subject; and
adjust the physical parameter of the numerical model based on the preliminary physiological data from the subject.

In this way, the numerical model may be tailored to the subject before the simulation begins, thereby increasing the accuracy of the determined hemodynamic function.

In an embodiment, the numerical model is based on a physical parameter and the method further comprises predicting a future hemodynamic function of the subject, wherein the processor is further adapted to:
adjust the physical parameter of the numerical model, thereby generating a predictive numerical model;
provide the continuous stream of physiological data as an input to the predictive numerical model, thereby simulating a predictive function of the cardiac system of the subject; and
predict the future hemodynamic function of the subject based on the simulated predictive function of the cardiac system of the subject.

In this way, a variety of different scenarios may be simulated that affect hemodynamic function in some way. For example, the effects of various stresses on the cardiac systems, or of various medications, may be investigated in the safety of the simulation.

In an embodiment, the physiological data comprises one or more of:
electrocardiogram data;
pressure numerical data;
pressure waveform data;
echocardiography-based physiology data; and
volume waveform data.

In a further embodiment, the volume waveform data comprises one or more of
a ventricular volume waveform; and
an atrial volume waveform.

In an embodiment, the volume waveform data comprises ultrasound data.

In an embodiment, the pressure waveform data comprises one or more of:
an atrial pressure waveform; and
an arterial pressure waveform.

In an embodiment, the physiological data comprises estimated physiological data.

In this way, intermittent data acquisition may be accounted for by providing estimated data.

According to another aspect of the invention, there is provided a computer-implemented method for determining a real-time valve function of a subject according to claim 14.

According to a further aspect of the invention, there is provided a computer program according to claim 15.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an ultrasound diagnostic imaging system to explain the general operation;
Figure 2 shows a method of the invention;
Figure 3 shows a schematic representation of a numerical model;
Figure 4 shows an example of a simulated hemodynamic function of a cardiac system; and
Figure 5 shows a schematic representation of a system for deriving a hemodynamic function of a cardiac system of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for determining a real-time valve function of a subject. The system comprises a processing unit adapted to: obtain a numerical model of a cardiac system, the numerical model being a 0D numerical model or a 1D numerical model, wherein the numerical model is adapted to receive physiological data as an input and output a simulated function of the cardiac system in real-time, wherein the simulated function of the cardiac system comprises a simulated function of a valve within the cardiac system. The processor is further adapted to obtain a continuous stream of physiological data from the subject; provide the continuous stream of physiological data as an input to the numerical model of the cardiac system, thereby generating a simulated function of the cardiac system of the subject; and determine a real-time valve function of the subject based on the simulated real-time function of the cardiac system of the subject.

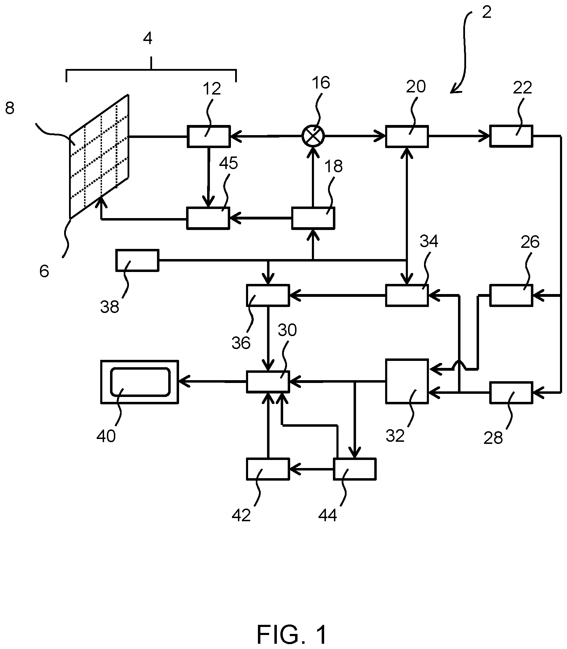

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise capacitive micromachined ultrasonic transducers (CMUTs); piezoelectric transducers, formed of materials such as PZT (lead zirconate titanate) or PVDF (polyvinylidene fluoride); or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The methods described herein may be performed on a processing unit. Such a processing unit may be located within an ultrasound system, such as the system described above with reference to Figure 1. For example, the image processor 30 described above may perform some, or all, of the method steps detailed below. Alternatively, the processing unit may be located in any suitable system, such as a monitoring system, that is adapted to receive an input relating to a subject.

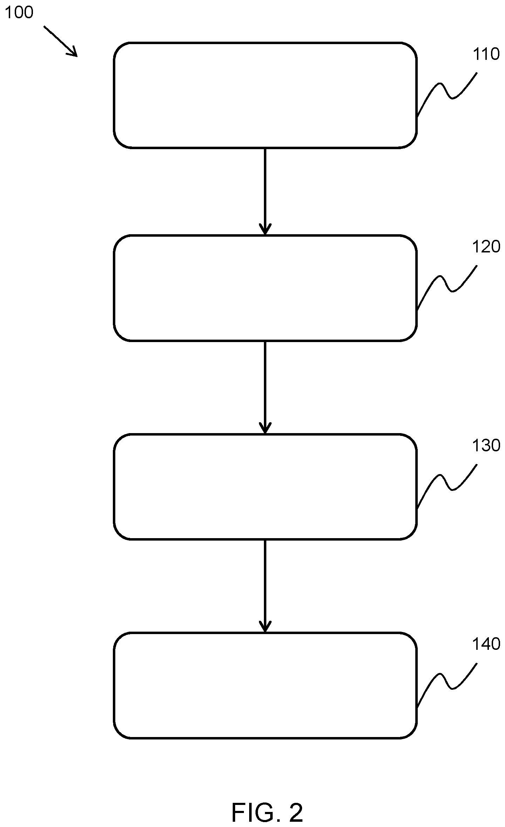

Figure 2 shows a method 100 for determining a real-time hemodynamic function of a subject. The real-time hemodynamic function may refer to any physical function where the movement, or flow, of blood is involved. For example, the real-time hemodynamic function may refer to the motion of blood passing through a valve, such as the mitral or aortic valve. Further, the real-time hemodynamic function may refer to the changes in volume of a given cardiac chamber, such as an atrium or ventricle.

The method begins in step 110 by obtaining a numerical model of a cardiac system, wherein the numerical model is adapted to take physiological data as an input and output a function of the cardiac system, the simulated function of the cardiac system comprising a simulated function of a valve within the cardiac system.

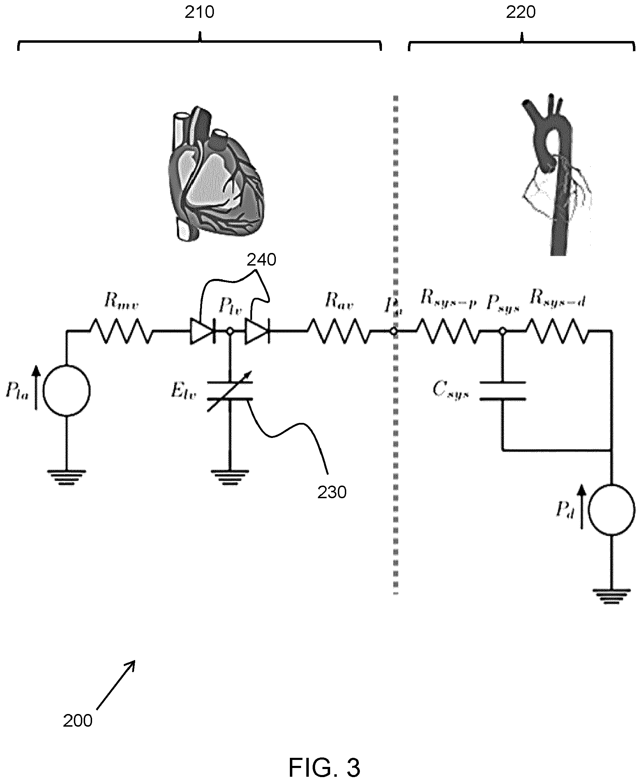

In other words, the numerical model simulates the function of a given cardiac system, such as a number of ventricles, atria and systemic arteries, a left side of the heart, an entire heart, and the like. For example, the cardiac system may include the left heart and the systemic arteries. An example of a numerical model for a cardiac system is described below with reference to Figure 3.

The numerical model may be constructed based on a variety of physical parameters. For example, the numerical model may be based on any one or more of: a pressure-volume relationship; a stiffness of the cardiac system; conservation of energy; conservation of mass; and conservation of momentum. The numerical model may also include a systemic arterial model, representing the arterial system of a subject.

The incorporation of physical parameters into the numerical model provides a framework within which data from various sources (such as: ultrasound data; peripheral, systemic or intra-cardiac blood pressure data; clinical guidelines; machine learning estimates; and the like) can be fused together following physical principals such as the conservation of mass, momentum and energy. Accordingly, a resultant estimate from the numerical model, such as a real-time hemodynamic function, may be made more consistent, particularly where inputs are noisy or are received from different imaging modalities and/or different moments in time are used.

Where the numerical model is based on one or more physical parameters, the method may further include adjusting the physical parameter(s) of the numerical model based on at least a portion of the continuous stream of physiological data. Alternatively, preliminary physiological data may be obtained from the subject and used to adjust the physical parameter(s) of the numerical model. Further, the physical parameter(s) of the numerical model may be adjusted based on physiological data collected from a number of subjects, i.e. using a population set of physiological data.

In other words, patient specific data may be used to tune the numerical model to an individual user. This may be performed during an examination, using the continuous stream of physiological data, or prior to the examination, using preliminary physiological data, which may be collected using the same sources as for the continuous stream of physiological data. By using patient-specific inputs (such as ultrasound volume segmentation taken from the ultrasound data or peripheral pressure measurements), the numerical model can be personalized to each patient, which in turn provides a patient-specific estimate of a cardiac function, for example real-time hemodynamic function.

The adjusting of the numerical model may include identifying a physical parameter based on the physiological data and providing the physical parameter to the numerical model.

For example, the physical parameter(s) may include one or more of: a systemic circulation parameter; a filling parameter; an ejection parameter; a heart rate parameter; a stiffness parameter; a valve related parameter, such as valve regurgitation or valve opening size; a blood flow parameter; and the like.

For example, an arterial blood pressure measurement may be used to tune the parameters of a systemic arterial model. The arterial blood pressure measurement may comprise one or more of a maximum, a minimum and a mean blood pressure value. The arterial blood pressure measurement may be used to adjust a resistance and a compliance of the systemic system in the numerical model, or vice versa.

In an example, a ventricular pressure measurement may be derived using an estimate of the pressure gradient over the aortic valve. The pressure gradient over the aortic valve may be estimated using Doppler ultrasound measurements. In the simplest implementation, the pressure gradient over the aortic valve may be assumed to be 0.

The adjustment of the numerical model may be further improved using a full blood pressure waveform. In this way, the numerical model may be further personalized to the subject, for example by including pressure decay constants in the simulation of the cardiac system, which may be used to model the stiffness and relaxation of a cardiac chamber.

Blood pressure information used to adjust the numerical model may be obtained from a peripheral artery, such as a brachial or radial artery, or a systemic artery such as the aortic or femoral artery. Measurements of peripheral pressures in such locations are amplified with respect to central aortic pressure values due to variations in vessel diameter and stiffness.

In the numerical model, the physical parameters may be identified using a multi-step approach. For example, physical parameters that represent the systemic circulation may be identified first (i.e. the parameters associated with the afterload of the cardiac system), before the parameters representing the ejection and filling of the heart are identified. These patient-specific physical parameters may be estimated using a mixture of techniques such as physiology based rules, direct optimization methods, sequential filtering methods and the like.

In step 120, a continuous stream of physiological data is obtained from the subject. The continuous stream of physiological data may be obtained from the subject in any suitable manner according to the application.

The physiological data may comprise one or more of: electrocardiogram data; pressure waveform data, which may include an atrial pressure waveform and/or an arterial pressure waveform; and volume waveform data, which may include a ventricular volume waveform and/or an atrial volume waveform. The volume waveform data may be based on ultrasound data collected from the subject. In addition, the physiological data comprises estimated physiological data, wherein the estimated physiological data is estimated based on available intermittent physiological data, thereby generating a continuous stream of physiological data based on the intermittent physiological data.

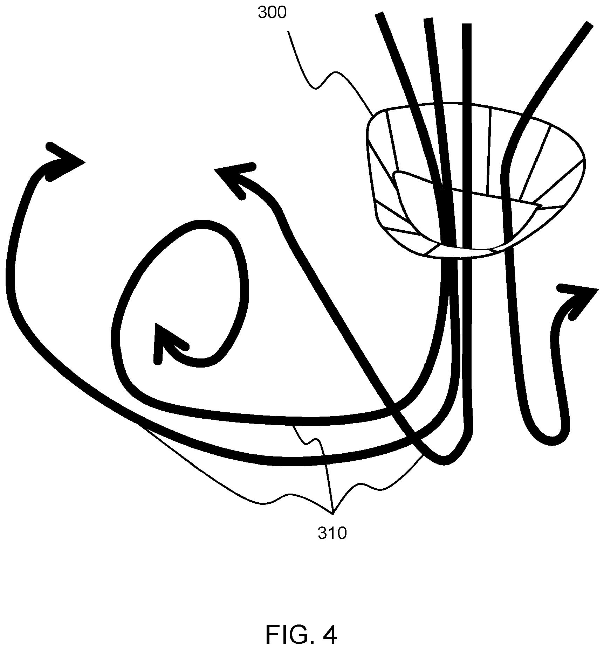

In step 130, the continuous stream of physiological data is provided as an input to the numerical model of the cardiac system, thereby simulating a real-time function of a cardiac system of the subject. An example of a simulation of a real-time function of a cardiac system is discussed further below with reference to Figure 4.

In step 140, the real-time hemodynamic function of the subject is determined based on the simulated real-time function of the cardiac system of the subject.

In patients undergoing MV repairs for MVR by a minimally invasive ETER, the goal is to reduce the level of MV regurgitation without significantly reducing impairing ventricular filling. In approximately 40% of ETERs this requires the use of multiple devices, such as clips, and the benefit of the second device is typically determined intra-operatively after the successful deployment of a first device. The current decision-making process primarily relies upon echocardiography assessment (i.e. qualitative anatomic inspection) or user-dependent measurements (such as, the pressure gradient over the MV).

However, there remain situations in which the benefit of an additional device remains unclear. In such subjects, the hemodynamic function (for example, the level of regurgitation vs. filling volumes at specific phases in the cardiac cycle) of the ETER may be further tested by altering the hemodynamic state of the subject pharmacologically to mimic a hemodynamic status more representative of normal conditions (i.e. post-intervention). Observing the hemodynamic response to these altered states enables the cardiologist to determine the suitability of the current ETER configuration.

The method described above may provide a real-time evaluation of hemodynamic function during an ETER procedure using a numerical model. Using patient-specific inputs (such as ultrasound volume segmentation, pressure waveforms, and the like.), such a model may be personalized on a beat-by-beat basis to each subject, providing a subject-specific estimate of the real-time hemodynamic function.

By way of example, the method may be deployed as follows. During ETER procedures, typically intermittent ultrasound is available, which would otherwise only provide a limited assessment of the hemodynamic function of the cardiac system of the subject. However, pressure waveforms, such as the left atrial and arterial pressures may be continuously recorded.

The numerical model may take the pressure waveforms and simultaneous volume waveforms (segmented from the ultrasound data when available) as an input and output a continuous estimate of the hemodynamic function of the subject. The volume waveforms may also be obtained by alternative techniques, such as using and inflatable finger cuff, thermodilution techniques, or by estimating complete volume waveforms based on available ultrasound data. The output of the numerical model may provide decision support on the effectiveness of deploying an additional repair device.

Furthermore, the numerical model may also produce the corresponding pressure-volume loops of the left heart (atrium and ventricle), thereby providing further physiological information for clinical assessment, such as the ability of the heart to adapt to altered conditions (such as exercise and/or stress).

In other words, the cardiac system may represent the heart, and specifically the left heart, of the subject and the method may further include determining a pressure-volume loop of a left ventricle and/or a left atrium of the cardiac system based on the simulated real-time function of the cardiac system of the subject.

In the methods described above, a real-time function of a valve of the subject is determined based on the real-time simulation generated from the continuous stream of physiological data. By real-time simulation, it is meant that there is little to no noticeable delay between the numerical model receiving the continuous stream of physiological data and the generation of the simulation of the cardiac system. Put another way, the real-time simulation of the cardiac system generates an output that is, or is almost, concurrent with the current state of the actual cardiac system of the subject. In this way, the system may provide an accurate real-time simulation of the functioning of the cardiac system that may be used during an interventional procedure, such as an edge-to-edge repair, in order to assess the progress of the procedure.

As it is not always possible, or desirable, to achieve an altered cardiac state pharmacologically, the numerical model may be employed to predict a future hemodynamic function of the subject. For example, a physical parameter of the numerical model, such as the ejection or regurgitation of a valve, may be adjusted according to a desired prediction scenario. The continuous stream of physiological data may then be provided as an input to the predictive numerical model, thereby simulating a predictive function of the cardiac system of the subject. Thus, the future hemodynamic function of the subject may be predicted based on the simulated predictive function of the cardiac system of the subject.

For example, during ETER procedures subjects are provided with anesthetic drugs, which temporarily reduce the vascular tone and therefore the afterload of the heart. Accordingly, the hemodynamic function of a subject as assessed during an ETER procedure may not be representative of normal situations. Accordingly, the numerical model may be used to predict the effect of an increased afterload virtually, thereby providing an estimate of hemodynamic function post-intervention. Thus, the numerical model may be used to provide additional decision support on the addition of a further repair device.

Put another way, the system for determining a real-time valve function of a subject may include one or more edge-to-edge valve repair devices and a processing unit adapted to implement the steps outlined above.

More specifically, the processing unit may be adapted to obtain a numerical model of a cardiac system, as described herein, and obtain a continuous stream of physiological data from the subject. The continuous stream of physiological data may be obtained from a subject undergoing a change in valve function, for example due to an edge-to-edge valve repair device being deployed at the valve.

The continuous stream of physiological data may be provided as an input to the numerical model of the cardiac system, thereby generating a simulated real-time function of the cardiac system of the subject and a real-time valve function of the subject may be determined based on the simulated real-time function of the cardiac system of the subject. The real-time valve function determined from the simulated real-time function of the cardiac system may be representative of the change in valve function.

Determining the real-time valve function of the subject may be performed by adjusting the simulated real-time function of the cardiac system based on a change in vascular tone of the cardiac system due to an anesthetic provided to the subject. Further, the numerical model may be further adapted to predict a future simulated valve function based on the provision of an additional edge-to-edge valve repair device to the valve based on the determined real-time valve function of the subject.

Figure 3 shows an example of a schematic representation of a numerical model 200 of a part of the cardiac system, namely, the left heart 210 and the aorta 220.

In this example, a simplified 0D approach to modelling blood flow, or hemodynamic function, during the heart cycle is represented. However, it is also possible to combine 1D/3D modelling approaches within the described framework and it is further possible to include a model of a complete circulatory system.

Zero dimensional (0D) models, also referred to as lumped-parameter models, give rise to a set of simultaneous ordinary differential equations (ODEs) describing the behavior of a cardiac system as discussed in detail in Shi, Y., Lawford, P. & Hose, R. "Review of Zero-D and 1-D Models of Blood Flow in the Cardiovascular System" BioMed Eng OnLine 10, 33 (2011), https://doi.org/10.1186/1475-925X-10-33. In representations of the vasculature, there are often two ODEs for each compartment, representing the conservation of mass and conservation of momentum, which may be complemented by an algebraic equilibrium equation relating compartment volume to pressure. Numerical models constructed from 0D components generally feature the major components of the cardiac system, such as the heart, the heart valves and compartments of the vasculature, and are suitable for the examination of global distributions of pressure, flow and blood volume over a range of physiological conditions.

One dimensional (1D), two dimensional (2D) and three dimensional (3D) models give rise to a series of partial differential equations describing conservation of mass and momentum (the Navier-Stokes equations), which may be complemented by algebraic equilibrium equations. In the context of cardiovascular numerical models, 1D models have the facility to represent wave transmission effects within the vasculature, which can be used to represent the aorta and larger systemic arteries. 3D models may be used to compute complex flow patterns, for example in the ventricles, around heart valves, near bifurcations, or in any region with vortical or separated flows. No analytical solutions are available for any 3D models but the simplest geometries, and recourse is always made to a numerical solution.

For example, in during an ETER procedure, the numerical model may be required to estimate a real-time hemodynamic function of a mitral valve of a subject. Figure 3 illustrates an example of a 0D numerical model 200, represented as an electronic circuit.

In carrying out 0D modelling, the concept of a hydraulic-electrical analog is often applied. Generally, there is a large amount of similarity between blood flow in the circulatory system and electric conduction in a circuit. For example, the blood pressure gradient in the circulatory loop drives the blood to flow against the hydraulic impedance and the voltage gradient in a circuit drives current to flow against the electric impedance. Hydraulic impedance represents the combined effect of the frictional loss, vessel wall elasticity and blood inertia in the blood flow, whilst electric impedance represents the combination of the resistance, capacitance and inductance in the circuit.

In the model shown in Figure 3, the voltage represents the blood pressure and the current represents the blood flow. In this approach the different compartments of the arterial system (such as the atria, ventricles, large arteries, and so on) are grouped together into electrical components which are related to hemodynamic analogues, such as resistance and capacitance.

Beginning with the left heart 210, the source (Pₗₐ) will charge the variable capacitor 230, mimicking the left atrium pumping blood to the left ventricle. The left atrium will fill the left ventricle (variable capacitor) to its passive limit. The physiological data obtained from the subject may include a continuous stream of left atrium pressure, which may be used to inform the model on the behavior of the left atrium.

The capacitance of the variable capacitor 230 represents the stiffness of the ventricle, i.e. the muscular contraction. The volume is a state variable that may be derived from the physiological data of the user.

Eₗᵥ refers to the elastance of the left ventricle. This relates the ventricular volume to the pressure within the left ventricle. Although it is a measure of the stiffness (i.e. muscular contraction of the ventricle), it is not strictly a material stiffness. This relationship has been experimentally measured from simultaneous ventricular pressure and volume waveforms.

The charge travels along the circuit, with the diodes 240 acting as valves to define a direction of flow. The blood (charge) then moves into the aorta 220 and enters the systemic circulation system.

The resistance terms (mitral valve resistance, Rₘᵥ, aortic valve resistance, Rₐᵥ, proximal systemic resistance, R_{sys=p}, distal systemic resistance, R_{sys=d}) represent the resistance of the blood vessels and valves to the blood flow and are directly related to the pressure in a given area. C_{sys} represents systemic compliance. The resistance terms may represent various stenoses within the cardiac system.

In this example, the model parameters are represented using electrical analogues. However, such models do follow physical principle such as conservation of mass, for example the blood flow (current) into each node of the model is conserved. Furthermore, the electrical analogue can be derived from the linearization of the mass and momentum conservation equation for blood flow in deformable vessels.

By taking a continuous stream of physiological data and an input (for example a combination of ultrasound data and pressure waveforms obtained from the subject) it is possible for the numerical model to output a representation of the real-time hemodynamic function of the cardiac system of the subject.

It should be noted that the example shown in Figure 3 is only one of many possible models of the left heart. The different components of the model may be interchanged dependent upon the specific application. In the example of an ETER procedure, the numerical model may be adapted to include a regurgitant mitral valve. Further, the model may be adapted to include a dynamic left atrium, dynamic left ventricle and the like. As described above, the numerical model may also include a systemic arterial model.

The model may be adapted to incorporate additional ultrasound data for specific applications, such as Doppler waveforms in heart diagnosis; however, this additional data must be routinely collected.

The numerical model described above may be integrated into an ultrasound analysis platform. This may enable the model to utilize the patient-specific segmentation of the left heart.

Put another way, in the example shown in Figure 3, a 0D numerical model is utilized to simulate the physics of blood flow in the heart. The numerical model is adapted to provide real-time patient-specific cardiac output or mitral regurgitation computations, such as a real-time hemodynamic function of the cardiac system of the subject. The input for this model may, for example, include quantitative echocardiography data of cardiac chambers (which may be based on ultrasound data obtained from the subject) and invasively, or non-invasively, measured pressure waves, relating to the blood pressure of the subject.

The numerical model may be a 0D numerical model or a 1D numerical model. As 0D and 1D numerical models are computationally simple, it is possible to process incoming physiological data from the subject with little to no delay, thereby providing a simulated function of the cardiac system in real-time.

In this model-based framework, pre-computed information, for example from complex 3D numerical models, may be provided. The pre-computed information may provide for an initial estimate for key physical parameters to be made, which may then be further personalized according to physiological data (for example, the non-linear valve resistance in ETER). The mapping of such non-linear pressure-flow relationships may be pre-computed over a range of different valve sizes and repair permutations using coupled fluid and solid numerical simulations. The pre-computed values may then be mapped to the patient based on the collected physiological data via a suitable technology (such as machine learning algorithms, proper orthogonal decomposition and the like).

In other words, computations having a high computational load may be performed before the application of the numerical model to the subject's physiological data, thereby reducing the computing resources and time required to simulate the cardiac system.

Figure 4 shows a visual representation of the simulated hemodynamic function of a repaired mitral valve 300 following an ETER procedure. In the example shown in Figure 4, the ribbons 310 represent the path of blood flow through the mitral valve and may be used to assess the hemodynamic function of the valve.

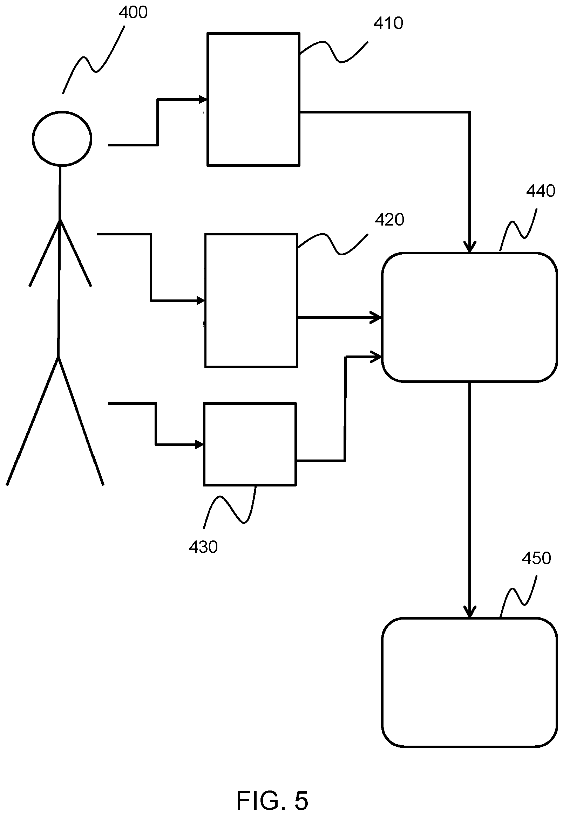

Figure 5 shows a schematic representation of a system for determining a real-time hemodynamic function of the subject 400.

In the example of an ETER procedure, the physiological data that may be available is shown below in Table 1.

**Table 1 - Data available in ETER**

| Data type | Continuous | Intermittent |
|---|---|---|
| Atrial pressure waveform | Yes | |
| Arterial pressure waveform | Yes | |
| ECG waveforms | Yes | |
| Ventricular volume waveform | Possible, dependent upon clinical situation | Yes |
| Atrial volume waveform | Possible, dependent upon clinical situation | Yes |

Referring to Figure 5, the ventricular volume waveform and the atrial volume waveform may be obtained by way of an ultrasound system 410. The arterial pressure waveform may be obtained by way of an anesthesiology set 420 and the atrial pressure waveform may be obtained by way of an interventional pressure measurement device 430.

Using the input data described above, the patient-specific physical parameters (such as resistance, compliance and the like) of the numerical model 440 may be estimated using, for example, a combination of physiological rules, direct optimization and sequential estimation. The numerical model may then output a real-time hemodynamic function 450 of the cardiac system of the subject, such as the mitral valve.

As detailed in Table 1, volume data, for example obtained by way of an ultrasound system, may be intermittent. Accordingly, the numerical model may need to operate for periods without volume waveform input. In this case, the physical parameters that govern the contractility of the heart (such as maximum contractility) may be used to automatically estimate the missing physiological data. The physiological data may be estimated, for example, by way of a combination of direct estimation techniques and non-linear state estimation (such as unscented Kalman filtering). When the volume waveform data is next available, the numerical model may then be updated using the new volume data, thereby recalibrating the numerical model.

In addition to an ultrasound system, volume waveform data may be obtained by way of an inflatable cuff, adapted to be worn by the subject and/or a thermistor-tipped catheter, wherein the volume waveform data is derived using a thermodilution technique.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A system for determining a real-time valve function of a subject (400), the system comprising:
a processing unit adapted to:
obtain a numerical model (210, 220) of a cardiac system, the numerical model being a 0D numerical model (210, 220), wherein the numerical model is represented as electronic circuit and is adapted to receive a continuous stream of physiological data as an input and to output a simulated hemodynamic function of the cardiac system in real-time, wherein the simulated function of the cardiac system comprises a simulated function of a valve within the cardiac system;
wherein within the model, voltage represents blood pressure, current represents blood flow, and model parameters are represented by electrical analogues;
obtain a continuous stream of physiological data from the subject;
provide the continuous stream of physiological data as an input to the numerical model of the cardiac system, thereby generating a simulated real-time function of the cardiac system of the subject; and
determine a real-time valve function of the subject based on the simulated real-time function of the cardiac system of the subject.

2. A system as claimed in claim 1, wherein the continuous stream of physiological data is obtained from a subject undergoing a change in valve function, and wherein the real-time valve function determined from the simulated real-time function of the cardiac system is representative of the change in valve function.

3. A system as claimed in claim 2, adapted for use while the subject is undergoing a valve repair.

4. A system as claimed in any of claims 1 to 3, wherein the system further comprises a physiological sensor adapted to obtain physiological data from the subject, wherein the physiological sensor comprises one or more of:
an electrocardiogram sensor, wherein the physiological data comprises electrocardiogram data;
a blood pressure measurement device, wherein the physiological data comprises numerical pressure data and/or pressure waveform data; and
a volume waveform sensor, wherein the physiological data comprises volume waveform data.

5. A system as claimed in claim 4, wherein the volume waveform sensor comprises one or more of:
an ultrasound transducer, wherein the volume waveform data comprises ultrasound data;
an inflatable cuff, adapted to be worn by the subject; and
a thermistor-tipped catheter, wherein the volume waveform data is derived using a thermodilution technique.

6. A system as claimed in any of claims 1 to 5, wherein the numerical model is based on a physical parameter, and wherein the processor is further adapted to adjust the physical parameter of the numerical model based on at least a portion of the continuous stream of physiological data.

7. A system as claimed in any of claims 1 to 6, wherein the numerical model is based on a physical parameter, and wherein the processor is further adapted to:
obtain preliminary physiological data from the subject; and
adjust the physical parameter of the numerical model based on the preliminary physiological data from the subject.

8. A system as claimed in any of claims 1 to 7, wherein the numerical model is based on a physical parameter, wherein the processor is further adapted to:
adjust the physical parameter of the numerical model, thereby generating a predictive numerical model;
provide the continuous stream of physiological data as an input to the predictive numerical model, thereby simulating a predictive function of the cardiac system of the subject; and
predict the future hemodynamic function of the subject based on the simulated predictive function of the cardiac system of the subject.

9. A system as claimed in any of claims 1 to 8, wherein the physiological data comprises one or more of:
electrocardiogram data;
pressure numerical data;
pressure waveform data;
echocardiography-based physiology data; and
volume waveform data.

10. A system as claimed in claim 9, wherein the volume waveform data comprises one or more of:
a ventricular volume waveform; and
an atrial volume waveform.

11. A system as claimed in any of claims 9 to 10, wherein the volume waveform data comprises ultrasound data.

12. A system as claimed in any of claims 9 to 11, wherein the pressure waveform data comprises one or more of:
an atrial pressure waveform; and
an arterial pressure waveform.

13. A system as claimed in any of claims 1 to 12, wherein the physiological data comprises estimated physiological data.

14. A computer-implemented method (100) for determining a real-time valve function of a subject (400), the method comprising:
obtaining (110) a numerical model (210, 220) of a cardiac system, the numerical model being a 0D numerical model (210, 220), wherein the numerical model is represented as electronic circuit and is adapted to receive a continuous stream of physiological data as an input and to output a simulated hemodynamic function of the cardiac system in real-time, wherein the simulated function of the cardiac system comprises a simulated function of a valve within the cardiac system;
wherein within the model, voltage represents blood pressure, current represents blood flow, and model parameters are represented by electrical analogues;
obtaining (120) a continuous stream of physiological data from the subject;
providing (130) the continuous stream of physiological data as an input to the numerical model of the cardiac system, thereby generating a simulated real-time function of the cardiac system of the subject; and
determining (140) a real-time valve function of the subject based on the simulated real-time function of the cardiac system of the subject.

15. A computer program comprising computer program code means which is adapted, when said computer program is run on a computer, to implement the method of claim 14.

## Patentansprüche

1. System zur Bestimmung einer Echtzeit-Klappenfunktion einer Person (400), wobei das System Folgendes umfasst:
eine Verarbeitungseinheit, die angepasst ist zum:
Erhalten eines numerischen Modells (210, 220) eines Herzsystems, wobei das numerische Modell ein numerisches 0D-Modell (210, 220) ist, wobei das numerische Modell als elektronischer Schaltkreis dargestellt ist und angepasst ist, um einen kontinuierlichen Strom physiologischer Daten als Eingabe zu empfangen und eine simulierte hämodynamische Funktion des Herzsystems in Echtzeit auszugeben, wobei die simulierte Funktion des Herzsystems eine simulierte Funktion einer Klappe innerhalb des Herzsystems umfasst;
wobei innerhalb des Modells die Spannung den Blutdruck darstellt und der Strom den Blutfluss darstellt und Modellparameter durch elektrische Analoga dargestellt werden;
Erhalten eines kontinuierlichen Stroms physiologischer Daten von der Person;
Bereitstellen des kontinuierlichen Stroms physiologischer Daten als Eingabe für das numerische Modell des Herzsystems, wodurch eine simulierte Echtzeitfunktion des Herzsystems der Person erzeugt wird; und
Bestimmen einer Echtzeit-Klappenfunktion der Person auf der Grundlage der simulierten Echtzeitfunktion des Herzsystems der Person.

2. System nach Anspruch 1, wobei der kontinuierliche Strom physiologischer Daten von einer Person erhalten wird, die eine Änderung der Klappenfunktion erfährt, und wobei die Echtzeit-Klappenfunktion, die aus der simulierten Echtzeitfunktion des Herzsystems bestimmt wird, repräsentativ für die Änderung der Klappenfunktion ist.

3. System nach Anspruch 2, das zur Verwendung angepasst ist, während sich die Person einer Klappenreparatur unterzieht.

4. System nach einem der Ansprüche 1 bis 3, wobei das System weiter einen physiologischen Sensor umfasst, der angepasst ist, um physiologische Daten von der Person zu erhalten, wobei der physiologische Sensor eines oder mehrere der Folgenden umfasst:
einen Elektrokardiogrammsensor, wobei die physiologischen Daten Elektrokardiogrammdaten umfassen;
eine Blutdruckmessvorrichtung, wobei die physiologischen Daten numerische Druckdaten und/oder Druckwellenformdaten umfassen; und
einen Volumenwellenformsensor, wobei die physiologischen Daten Volumenwellenformdaten umfassen.

5. System nach Anspruch 4, wobei der Volumenwellenformsensor eines oder mehrere der Folgenden umfasst:
einen Ultraschallwandler, wobei die Volumenwellenformdaten Ultraschalldaten umfassen;
eine aufblasbare Manschette, die angepasst ist, um von der Person getragen zu werden; und
einen Katheter mit Thermistorspitze, wobei die Volumenwellenformdaten unter Verwendung einer Thermodilutionstechnik abgeleitet werden.

6. System nach einem der Ansprüche 1 bis 5, wobei das numerische Modell auf der Grundlage eines physikalischen Parameters aufgebaut ist und wobei der Prozessor weiter angepasst ist, um den physikalischen Parameter des numerischen Modells auf der Grundlage von mindestens einem Teil des kontinuierlichen Stroms physiologischer Daten anzupassen.

7. System nach einem der Ansprüche 1 bis 6, wobei das numerische Modell auf der Grundlage eines physikalischen Parameters aufgebaut ist und wobei der Prozessor weiter angepasst ist zum:
Erhalten vorläufiger physiologischer Daten von der Person; und
Anpassen des physikalischen Parameters des numerischen Modells auf der Grundlage der vorläufigen physiologischen Daten der Person.

8. System nach einem der Ansprüche 1 bis 7, wobei das numerische Modell auf der Grundlage eines physikalischen Parameters aufgebaut ist, wobei der Prozessor weiter angepasst ist zum:
Anpassen des physikalischen Parameters des numerischen Modells, wodurch ein prädiktives numerisches Modell erzeugt wird;
Bereitstellen des kontinuierlichen Stroms physiologischer Daten als Eingabe für das prädiktive numerische Modell, wodurch eine prädiktive Funktion des Herzsystems der Person simuliert wird; und
Vorhersagen der zukünftigen hämodynamischen Funktion der Person auf der Grundlage der simulierten prädiktiven Funktion des Herzsystems der Person.

9. System nach einem der Ansprüche 1 bis 8, wobei die physiologischen Daten eines oder mehrere der Folgenden umfassen:
Elektrokardiogrammdaten;
numerische Druckdaten;
Druckwellenformdaten;
physiologische Daten auf der Grundlage der Echokardiographie; und
Volumenwellenformdaten.

10. System nach Anspruch 9, wobei die Volumenwellenformdaten eines oder mehrere der Folgenden umfassen:
eine Wellenform des ventrikulären Volumens; und
eine Wellenform des atrialen Volumens.

11. System nach einem der Ansprüche 9 bis 10, wobei die Volumenwellenformdaten Ultraschalldaten umfassen.

12. System nach einem der Ansprüche 9 bis 11, wobei die Druckwellenformdaten eines oder mehrere der Folgenden umfassen:
eine Wellenform des atrialen Drucks; und
eine Wellenform des arteriellen Drucks.

13. System nach einem der Ansprüche 1 bis 12, wobei die physiologischen Daten geschätzte physiologische Daten umfassen.

14. Computerimplementiertes Verfahren (100) zur Bestimmung einer Echtzeit-Klappenfunktion einer Person (400), wobei das Verfahren Folgendes umfasst:
Erhalten (110) eines numerischen Modells (210, 220) eines Herzsystems, wobei das numerische Modell ein numerisches 0D-Modell (210, 220) ist, wobei das numerische Modell als elektronischer Schaltkreis dargestellt ist und angepasst ist, um einen kontinuierlichen Strom physiologischer Daten als Eingabe zu empfangen und eine simulierte hämodynamische Funktion des Herzsystems in Echtzeit auszugeben, wobei die simulierte Funktion des Herzsystems eine simulierte Funktion einer Klappe innerhalb des Herzsystems umfasst;
wobei innerhalb des Modells die Spannung den Blutdruck darstellt und der Strom den Blutfluss darstellt und Modellparameter durch elektrische Analoga dargestellt werden;
Erhalten (120) eines kontinuierlichen Stroms physiologischer Daten von der Person;
Bereitstellen (130) des kontinuierlichen Stroms physiologischer Daten als Eingabe für das numerische Modell des Herzsystems, wodurch eine simulierte Echtzeitfunktion des Herzsystems der Person erzeugt wird; und
Bestimmen (140) einer Echtzeit-Klappenfunktion der Person auf der Grundlage der simulierten Echtzeitfunktion des Herzsystems der Person.

15. Computerprogramm, umfassend Computerprogrammcodemittel, die angepasst sind, wenn das Computerprogramm auf einem Computer läuft, das Verfahren nach Anspruch 14 zu implementieren.

## Revendications

1. Système pour déterminer une fonction de valve en temps réel d'un sujet (400), le système comprenant :
une unité de traitement adaptée pour :
obtenir un modèle numérique (210, 220) d'un système cardiaque, le modèle numérique étant un modèle numérique 0D (210, 220), dans lequel le modèle numérique est représenté sous la forme d'un circuit électronique et est adapté pour recevoir un flux continu de données physiologiques en tant qu'entrée et pour délivrer en sortie une fonction hémodynamique simulée du système cardiaque en temps réel, dans lequel la fonction simulée du système cardiaque comprend une fonction simulée d'une valve à l'intérieur du système cardiaque ;
dans lequel, dans le modèle, la tension représente la pression artérielle, le courant représente le flux sanguin et des paramètres du modèle sont représentés par des analogues électriques ;
obtenir un flux continu de données physiologiques du sujet ;
fournir le flux continu de données physiologiques en tant qu'entrée au modèle numérique du système cardiaque, ce qui permet de générer une fonction simulée en temps réel du système cardiaque du sujet ; et
déterminer une fonction de valve en temps réel du sujet sur la base de la fonction en temps réel simulée du système cardiaque du sujet.

2. Système selon la revendication 1, dans lequel le flux continu de données physiologiques est obtenu d'un sujet subissant un changement de fonction de valve et dans lequel la fonction de valve en temps réel déterminée à partir de la fonction en temps réel simulée du système cardiaque est représentative du changement de fonction de valve.

3. Système selon la revendication 2, adapté pour être utilisé pendant que le sujet subit une réparation de valve.

4. Système selon l'une quelconque des revendications 1 à 3, dans lequel le système comprend en outre un capteur physiologique adapté pour obtenir des données physiologiques du sujet, dans lequel le capteur physiologique comprend un ou plusieurs :
d'un capteur d'électrocardiogramme, dans lequel les données physiologiques comprennent des données d'électrocardiogramme ;
d'un dispositif de mesure de pression artérielle, dans lequel les données physiologiques comprennent des données de pression numériques et/ou des données de forme d'onde de pression ; et
d'un capteur de forme d'onde de volume, dans lequel les données physiologiques comprennent des données de forme d'onde de volume.

5. Système selon la revendication 4, dans lequel le capteur de forme d'onde de volume comprend un ou plusieurs :
d'un transducteur à ultrasons, dans lequel les données de forme d'onde de volume comprennent des données ultrasonores ;
d'un brassard gonflable, adapté pour être porté par le sujet ; et
d'un cathéter à pointe de thermistance, dans lequel les données de forme d'onde de volume sont dérivées à l'aide d'une technique de thermodilution.

6. Système selon l'une quelconque des revendications 1 à 5, dans lequel le modèle numérique est basé sur un paramètre physique et dans lequel le processeur est en outre adapté pour ajuster le paramètre physique du modèle numérique sur la base d'au moins une partie du flux continu de données physiologiques.

7. Système selon l'une quelconque des revendications 1 à 6, dans lequel le modèle numérique est basé sur un paramètre physique et dans lequel le processeur est en outre adapté pour :
obtenir des données physiologiques préliminaires du sujet ; et
ajuster le paramètre physique du modèle numérique en fonction des données physiologiques préliminaires du sujet.

8. Système selon l'une quelconque des revendications 1 à 7, dans lequel le modèle numérique est basé sur un paramètre physique, dans lequel le processeur est en outre adapté pour :
ajuster le paramètre physique du modèle numérique, ce qui permet de générer un modèle numérique prédictif ;
fournir le flux continu de données physiologiques en tant qu'entrée au modèle numérique prédictif, ce qui permet de simuler une fonction prédictive du système cardiaque du sujet ; et
prédire la future fonction hémodynamique du sujet en fonction de la fonction prédictive simulée du système cardiaque du sujet.

9. Système selon l'une quelconque des revendications 1 à 8, dans lequel les données physiologiques comprennent une ou plusieurs :
de données d'électrocardiogramme ;
de données numériques de pression ;
de données de forme d'onde de pression ;
de données physiologiques basées sur une échocardiographie ; et
de données de forme d'onde de volume.

10. Système selon la revendication 9, dans lequel les données de forme d'onde de volume comprennent une ou plusieurs :
d'une forme d'onde de volume ventriculaire ; et
d'une forme d'onde de volume auriculaire.

11. Système selon l'une quelconque des revendications 9 à 10, dans lequel les données de forme d'onde de volume comprennent des données ultrasonores.

12. Système selon l'une quelconque des revendications 9 à 11, dans lequel les données de forme d'onde de pression comprennent une ou plusieurs :
d'une forme d'onde de pression auriculaire ; et
d'une forme d'onde de pression artérielle.

13. Système selon l'une quelconque des revendications 1 à 12, dans lequel les données physiologiques comprennent des données physiologiques estimées.

14. Procédé mis en oeuvre par ordinateur (100) pour déterminer une fonction de valve en temps réel d'un sujet (400), le procédé comprenant :
l'obtention (110) d'un modèle numérique (210, 220) d'un système cardiaque, le modèle numérique étant un modèle numérique 0D (210, 220), dans lequel le modèle numérique est représenté sous la forme d'un circuit électronique et est adapté pour recevoir un flux continu de données physiologiques en tant qu'entrée et pour délivrer en sortie une fonction hémodynamique simulée du système cardiaque en temps réel, dans lequel la fonction simulée du système cardiaque comprend une fonction simulée d'une valve à l'intérieur du système cardiaque ;
dans lequel, dans le modèle, la tension représente la pression artérielle, le courant représente le flux sanguin et des paramètres du modèle sont représentés par des analogues électriques ;
l'obtention (120) d'un flux continu de données physiologiques du sujet ;
la fourniture (130) du flux continu de données physiologiques en tant qu'entrée au modèle numérique du système cardiaque, ce qui permet de générer une fonction simulée en temps réel du système cardiaque du sujet ; et
la détermination (140) d'une fonction de valve en temps réel du sujet sur la base de la fonction en temps réel simulée du système cardiaque du sujet.

15. Programme informatique comprenant un moyen de code de programme informatique qui est adapté, lorsque ledit programme informatique est exécuté sur un ordinateur, pour mettre en oeuvre le procédé de la revendication 14.
